**Europäisches Patentamt**

**European Patent Office**

**Office européen des brevets**

(19)

(11) Veröffentlichungsnummer : **0 479 874 B1**

(12) # EUROPÄISCHE PATENTSCHRIFT

(45) Veröffentlichungstag der Patentschrift :
27.10.93 Patentblatt 93/43

(51) Int. Cl.$^5$ : **A61K 31/275**

(21) Anmeldenummer : **90910642.9**

(22) Anmeldetag : **27.06.90**

(86) Internationale Anmeldenummer :
**PCT/EP90/01031**

(87) Internationale Veröffentlichungsnummer :
**WO 91/00091 10.01.91 Gazette 91/02**

(54) **VERWENDUNG VON NOR-VERAPAMIL UND NOR-GALLOPAMIL ALS ANTIARTERIOSKLEROTICA.**

(30) Priorität : **27.06.89 DE 3921006**

(43) Veröffentlichungstag der Anmeldung :
**15.04.92 Patentblatt 92/16**

(45) Bekanntmachung des Hinweises auf die
Patenterteilung :
**27.10.93 Patentblatt 93/43**

(84) Benannte Vertragsstaaten :
**AT BE CH DE FR GB IT LI NL**

(56) Entgegenhaltungen :
**EP-A- 0 075 823**
**EP-A- 0 285 906**
**The Am. J. of Cardiology, vol. 55, 1985, W.W.**
**Parmley et al., pp. 165B-171B**
**Cardiovascular Research, vol. 12, 1978, G.**
**Neugebauer, pp. 247-254**

(73) Patentinhaber : **KNOLL AG**
**Postfach 21 08 05**
**D-67061 Ludwigshafen (DE)**

(72) Erfinder : **MUELLER, Claus, D.**
**Im Schaflaeger 30**
**D-6806 Viernheim (DE)**
Erfinder : **UNGER, Liliane**
**Waltraudenstrasse 14**
**D-6700 Ludwigshafen (DE)**

(74) Vertreter : **Karau, Wolfgang, Dr. et al**
**BASF Aktiengesellschaft, Patentabteilung**
**ZDX - C 6**
**D-67056 Ludwigshafen (DE)**

EP 0 479 874 B1

## Beschreibung

Die Erfindung betrifft die Verwendung von Nor-Verapamil (5-[N-(3,4-Dimethoxyphenethyl)-amino]-2-(3,4-dimethoxyphenyl)-2-isopropyl-Valeronitril) und Nor-Gallopamil(5-[N-(3,4-Dimethoxyphenethyl)-amino]-2-(3,4,5-trimethoxyphenethyl)-2-isopropyl-valeronitril) zur Herstellung von Arzneimitteln mit antiarteriosklerotischen Eigenschaften.

Nor-Verapamil ist aus der EP-OS 285 906 und der Veröffentlichung G. Neugebauer, Cardiovasc. Rs 1978, 12 (4) 247 - 254, bekannt.

Nor-Gallopamil ist von M. Nieder und H. Jaeger in Journal of Chromatography 1987, 414, 492 - 498, beschrieben.

Weiter ist aus der DE-PS 1 154 810 das Verapamil (5-[N-(3,4-Dimethoxyphenethyl)-N-methylaminol-2-(3,4-dimethoxyphenyl)-2-isopropyl-valeronitril) und das Gallopamil (5-[N-(3,4-Dimethoxyphenethyl)-N-methylamino]-2-(3,4,5-trimethoxyphenyl]-2-isopropyl-valeronitril) bekannt. Die Wirkung von Verapamil gegen Arteriosklerose ist untersucht worden (JACC 1, 1453, 1983, Am. J. Cardiol. 54, 884, 1984).

Es wurde jetzt gefunden, daß Nor-Verapamil und Nor-Gallopamil eine sehr gute Wirkung gegen Arteriosklerose besitzen.

Gegenstand der Erfindung ist die Verwendung von Nor-Verapamil und Nor-Gallopamil und deren Salzen mit physiologisch verträglichen Säuren zur Herstellung von Arzneimitteln mit antiarteriosklerotischen Eigenschaften.

Gegenstand der Erfindung sind weiter Nor-Gallopamil und dessen Salze mit physiologisch verträglichen Säuren zur Verwendung bei der Bekämpfung von Krankheiten.

Nor-Verapamil und Nor-Gallopamil können in freier Form angewendet werden. Zweckmäßigerweise werden sie jedoch in Form eines Salzes mit einer physiologisch verträglichen Säure verwendet (vgl. DE-PS 1 154 810).

Arteriosklerose ist eine häufig auftretende, auf multifaktoriellen Ursachen beruhende Erkrankung der arteriellen Gefäßwand, in deren Folge es durch Bildung von Atheromen zur Einengung der Strombahn und zur Gefäßverschlüssen kommt. Die wichtigsten für die Atherombildung verantwortlichen Faktoren sind:
- eine Erhöhung der Permeabilität des Aortenendothels für Makromoleküle (Lipoproteine)
- Migration glatter Muskelzellen aus dem Verband der Gefäßintima mit anschließender Proliferation und Synthese von Grundsubstanzen (Bildung arteriosklerotischer Plaques)
- Einwanderung von Makrophagen, welche die übermäßig eindringenden Lipoproteine aufnehmen (Schaumzellbildung).

Mit den erfindungsgemäßen Verbindungen kann die Bildung von Atheromen in der Gefäpwand und das Fortschreiten arteriosklerotischer Veränderungen gehemmt bzw. deren Regression gefördert werden.

Zum Nachweis der antiarteriosklerotischen Wirkung werden die Substanzen männlichen Kaninchen über 28 Tage täglich 2mal subkutan und 1mal oral im Abstand von jeweils 4 h appliziert. Lokale Atherome werden durch elektrische Reizung der A. carotis bei gleichzeitiger Fütterung mit cholesterinreicher Diät (2 %) nach der Methode von E. Betz und W. Schlote (Basic Res. Cardiol. 1979, 74, 10 - 20) induziert. Am Ende der Versuchsperiode werden die unter der Anode der Reizelektroden gebildeten Atherome entnommen und deren Gröpe histologisch ermittelt. Als Maß für die Größe der Atherome dient die maximale Zahl ihrer Zellagen, die mikroskopisch aus Serienschnitten ermittelt wird. Das Ausmap der antiarterisklerotischen Wirkung wird durch Vergleich mit unbehandelten Kontrolltieren festgestellt. Als Vergleichssubstanzen dienen Verapamil und Gallopamil.

In diesem Test hemmen Nor-Verapamil und Nor-Gallopamil die Entwicklung von Atheromen etwa im gleichen Ausmaß wie Verapamil. Gallopamil ist in der höchsten tolerierten Tagesdosis ohne antiatherogenen Effekt (Tabelle 1). Die Ca-antagonistische Wirkungsstärke - bestimmt aus der Bindung an den Ca-Kanal durch Verdrängung der spezifischen (S)-$^3$H-Devapamil-Bindung in Membranpräparationen von Meerschweinchen-Skelettmuskeln (Tabelle 2) - ist um den Faktor 8 bzw. 10 geringer als bei Verapamil bzw. Gallopamil. Durch ihre schwache Ca-antagonistische Wirkung sind die erfindungsgemäßen Verbindungen dem Verapamil bei der Behandlung von Arteriosklerose deutlich überlegen, da sie bei gleicher und höherer Dosierung keine kardiovaskulären Nebenwirkungen hervorrufen.

Nor-Verapamil und Nor-Gallopamil bzw. deren Salze können üblicherweise oral oder parenteral verabreicht werden. Die Dosierung hängt vom Alter, Zustand und Gewicht des Patienten sowie von der Applikationsart ab. In der Regel beträgt die tägliche Wirkstoffdosis etwa 1 bis 30, vorzugsweise 5 bis 25 mg/kg Körpergewicht bei oraler oder rektaler Gabe und 0,05 - 5, vorzugsweise 0,5 bis 3 mg/kg Körpergewicht bei parenteraler Gabe.

Die Substanzen weisen ein Asymmetriezentrum auf und sind zur Spaltung in Enantiomere geeignet.

Gegebenenfalls werden die erhaltenen erfindungsgemäpen Verbindungen in das Säureadditionssalz einer physiologisch verträglichen Säure überführt. Eine Zusammenstellung üblicher physiologisch verträglicher Säuren kann aus Fortschritte der Arzneimittelforschung 1966, Deutschland, Schweiz, Birkhäuser Verlag, Bd. 10, S. 224-285 und J. Pharm. Sci., Bd. 66 (1977), S. 1-5 entnommen werden. Bevorzugt ist Salzsäure.

Die Säureadditionssalze werden in der Regel in an sich bekannter Weise durch Mischen der freien Base oder deren Lösungen mit der entsprechenden Säure oder deren Lösungen in einem organischen Lösungsmittel, beispielsweise einem niederen Alkohol, wie Methanol, Ethanol oder Propanol, oder einem niederen Keton, wie Aceton, Methylethylketon oder Methylisobutylketon, oder einem Ether, wie Diethylether, Tetrahydrofuran oder Dioxan erhalten. Zur besseren Kristallabscheidung können Mischungen der genannten Lösungsmittel verwendet werden. Darüber hinaus können pharmazeutisch vertretbare wäßrige Lösungen von Säure-Additionsverbindungen der Aminoverbindungen der Formel I durch Auflösen der freien Basen in einer wäßrigen Säurelösung hergestellt werden.

Nor-Verpamil und Nor-Gallopamil und deren Salze können in den gebräuchlichen galenischen Applikationsformen fest oder flüssig angewendet werden, z.B. in Form von Tabletten, Filmtabletten, Kapseln, Pulvern, Granulaten, Dragees, Suppositorien, Lösungen oder Dosieraerosolen. Diese werden in der üblichen Weise hergestellt. Die Wirkstoffe können dabei mit den gebräuchlichen galenischen Hilfsmitteln wie Tablettenbindern, Füllstoffen, Konservierungsmitteln, Tablettensprengmitteln, Fließregulierungsmitteln, Weichmachern, Netzmitteln, Dispergiermitteln, Emulgatoren, Lösungsmitteln, Retardierungsmitteln und/oder Antioxidantien verarbeitet werden (vgl. H. Sucker et al: Pharmazeutische Technologie, Thieme-Verlag, Stuttgart Weichmachern, Netzmitteln, Dispergiermitteln, Emulgatoren, Lösungsmitteln, Retardierungsmitteln und/oder Antioxidantien verarbeitet werden (vgl. H. 1978). Die so erhaltenen Zubereitungen enthalten den Wirkstoff normalerweise in einer Menge von 0,1 bis 99 Gew.-%.

Beispiele 1 und 2

Auf einer Tablettenpresse werden in üblicher Weise Tabletten folgender Zusammensetzung geprept:

| 40 mg | Nor-Verapamil-Hydrochlorid (Bsp. 1) bzw. Nor-Gallopamil-Hydrochlorid (Bsp. 2) |
| 120 mg | Maisstärke |
| 13,5 mg | Gelatine |
| 45 mg | Milchzucker |
| 2,25 mg | Aerosil® (chemisch reine Kieselsäure in submikroskopisch feiner Verteilung |
| 6,75 mg | Kartoffelstärke (als 6%iger Kleister) |

Beispiele 3 und 4

In üblicher Weise werden Dragees folgender Zusammensetzung hergestellt:

| 20 mg | Nor-Verapamil-Hydrochlorid (Bsp. 3) bzw. |
|  | Nor-Gallopamil-Hydrochlorid (Bsp. 4) |
| 60 mg | Kernmasse |
| 60 mg | Verzuckerungsmasse. |

Die Kernmasse besteht aus 9 Teilen Maisstärke, 3 Teilen Milchzucker und 1 Teil Vinylpyrrolidon-Vinylacetat-Mischpolymerisat 60:40 (vgl. Pharm. Ind. 1962, 586). Die Verzuckerungsmasse besteht aus 5 Teilen Rohrzucker, 2 Teilen Maisstärke, 2 Teilen Calciumcarbonat und 1 Teil Talk. Die so hergestellten Dragees werden anschließend mit einem magensaftresistenten überzug versehen.

Beispiele 5 und 6

10 g Nor-Verapamil-Hydrochlorid (Bsp. 5) bzw. Nor-Gallopamil-Hydrochlorid (Bsp. 6) werden in 5000 ml Wasser unter Zusatz von NaCl gelöst und mit 0,1 normaler NaOH auf pH 6,0 eingestellt, so daß eine blutisotonische Lösung entsteht. Jeweils 5 ml dieser Lösung werden in Ampullen gefüllt und sterilisiert.

Tabelle 1 - Antiatherogene Wirkung am Kaninchen

| Substanz | Tagesdosis mg/kg | | Tierzahl | Maximale Zellagenzahl der Atherome | |
|---|---|---|---|---|---|
| | s.c. | per os | N | n | Änderung % |
| Verapamil | – | – | 5 | $7,8 \pm 1,0$ | |
| | 2 x 1,0 | 21,5 | 5 | $3,8 \pm 0,37*$ | – 51 |
| Nor-Verapamil | – | – | 8 | $14 \pm 2,4$ | |
| | 2 x 1,0 | 21,5 | 8 | $8,1 \pm 1,3$ 1) | – 40 |
| Gallopamil | – | – | 9 | $14 \pm 1,2$ | |
| | 2 x 0,215** | 4,64** | 9 | $15 \pm 0,99$ | + 7,1 |
| Nor-Gallopamil | – | – | 6 | $22 \pm 3,2$ | |
| | 2 x 1,0 | 21,5 | 7 | $12 \pm 1,3*$ | – 44 |

*) P < 0,05 1) P = 0,682      **) höchste tolerierte Tagesdosis

Tabelle 2 - Bindung an den Kalzium-Kanal

| Substanz | Ki (nM) |
|---|---|
| | $(S)-^3H$-Devapamilverdrängung |
| Verapamil | 41 |
| Nor-Verapamil | 320 |
| Gallopamil | 24 |
| Nor-Gallopamil | 247 |

Membranhomogenat wird mit steigenden Konzentrationen ($10^{-10}$ - $10^{-6}$ M) von Prüfsubstanz und einer festen Konzentration von 1 nM Radioligand 60 min. bei Raumtemperatur inkubiert. Der gebundene und der freie Radioligand werden durch Filtration über Glasfaserfilter getrennt und die Menge des auf dem Filter zurückgehaltenen Radioliganden mittels Flüssigkeitsszintillationsmessung bestimmt. Es werden 2 Versuche in 3fachem Ansatz durchgeführt.

Die Kompetitionskonstante ($K_i$-Werte in nM) wird durch nichtlineare Regressionsanalyse an einem IBM-Rechner in Anlehnung an das Programm Ligand von Munson und Rodboard (Analytical Biochemistry 107, 220, 1980) berechnet.

**Patentansprüche**

1. Verwendung von Nor-Verapamil oder Nor-Gallopamil und deren Salzen mit physiologisch verträglichen Säuren zur Herstellung von Arzneimitteln mit antiarteriosklerotischen Eigenschaften.

2. Arzneimittel, das neben üblichen galenischen Hilfsmitteln Nor-Gallopamil als Wirkstoff enthält.

**Claims**

1.  The use of nor-verapamil or nor-gallopamil and salts thereof with physiologically tolerated acids for producing drugs with antiarteriosclerotic properties.

2.  A drug which, besides conventional pharmaceutical aids, contains nor-gallopamil as active substance.

**Revendications**

1.  Emploi de nor-vérapamil ou de nor-gallopamil ainsi que leurs sels avec des acides acceptables sur le plan physiologique pour la préparation de médicaments ayant des propriétés anti-artérioscléreuses.

2.  Médicament qui contient à côté des adjuvants galéniques habituels du nor-gallopamil comme substance active.